# EUROPEAN PATENT APPLICATION

(11) **EP 1 944 323 A1**
(43) Date of publication of application: **16.07.2008**
(21) Application number: 06810358.9
(22) Date of filing: 14.09.2006
(51) Int. Cl.: C07K 16/30, C12N 15/09, G01N 33/53, G01N 33/577, C12P 21/08

(54) **NOVEL MONOCLONAL ANTIBODY AND USE THEREOF**

(30) Priority: 15.09.2005 JP 2005269072
(71) Applicant: OSAKA UNIVERSITY, Suita-shi, Osaka 565-0871 (JP); Takeda Pharmaceutical Company Limited, Osaka-shi, Osaka 541-0045 (JP)
(72) Inventor: HASHIMOTO, Jun, Suita-shi Osaka 565-0871 (JP); NAMPEI, Akihide, Suita-shi Osaka 565-0871 (JP); ANDO, Wataru, Suita-shi Osaka 565-0871 (JP); KUROKAWA, Tomofumi, Osaka-shi Osaka 532-8686 (JP); OSHIMA, Tsutomu, Osaka-shi Osaka 532-8686 (JP)
(74) Representative: Jones, Nicholas Andrew
(86) International application number: PCT/JP2006/318678
(87) International publication number: WO 2007/032560

(57) **Abstract**

The present invention provides an antibody capable of specifically recognizing the same amino acid sequence as an antigenic determinant of mouse monoclonal antibody MAB-ME-16F4.3 (FERM BP-10329), and an antibody of capable of specifically recognizing the same amino acid sequence as an antigenic determinant of mouse monoclonal antibody MAB-ME-12C9.2 (FERM BP-10328), and methods of risk prediction of bone fracture and/or diagnosis of osteoporosis by detecting a MEPE-derived molecule in a biological sample using the above-mentioned two kinds of antibodies.

SEQUENCE LISTING

<110>  OSAKA UNIVERSITY
       Takeda Pharmaceutical Company Limited

<120>  Novel Monoclonal Antibody and Use Thereof

<130>  09969

<150>  JP 2005-269072
<151>  2005-09-15

<160>  7

<170>  PatentIn version 3.2

<210>  1
<211>  1575
<212>  DNA
<213>  Homo sapiens


<220>
<221>  CDS
<222>  (1)..(1575)

<400>  1
atg cga gtt ttc tgt gtg gga cta ctc ctt ttc agt gtg acc tgg gca        48
Met Arg Val Phe Cys Val Gly Leu Leu Leu Phe Ser Val Thr Trp Ala
1               5                   10                  15

gca cca aca ttt caa cca cag act gag aaa act aag caa agc tgt gtg        96
Ala Pro Thr Phe Gln Pro Gln Thr Glu Lys Thr Lys Gln Ser Cys Val
            20                  25                  30

gaa gag cag agg cag gaa gaa aaa aac aaa gac aat att ggt ttt cac       144
Glu Glu Gln Arg Gln Glu Glu Lys Asn Lys Asp Asn Ile Gly Phe His
        35                  40                  45

cat ttg ggc aag aga ata aat caa gag cta tca tct aaa gaa aat att       192
His Leu Gly Lys Arg Ile Asn Gln Glu Leu Ser Ser Lys Glu Asn Ile
    50                  55                  60

gtc cag gaa aga aag aaa gat ttg tcc ctt tct gaa gcc agt gag aat       240
Val Gln Glu Arg Lys Lys Asp Leu Ser Leu Ser Glu Ala Ser Glu Asn
65                  70                  75                  80

aag gga agt agt aaa tct caa aat tat ttc aca aat aga cag aga ctg       288
Lys Gly Ser Ser Lys Ser Gln Asn Tyr Phe Thr Asn Arg Gln Arg Leu
                85                  90                  95

aat aaa gaa tat agt atc agt aac aaa gag aat act cac aat ggc ctg       336
Asn Lys Glu Tyr Ser Ile Ser Asn Lys Glu Asn Thr His Asn Gly Leu
            100                 105                 110

agg atg tca att tat cct aag tca act ggg aat aaa ggg ttt gag gat       384
Arg Met Ser Ile Tyr Pro Lys Ser Thr Gly Asn Lys Gly Phe Glu Asp
        115                 120                 125

gga gat gat gct atc agc aaa cta cat gac caa gaa gaa tat ggc gca       432
Gly Asp Asp Ala Ile Ser Lys Leu His Asp Gln Glu Glu Tyr Gly Ala
    130                 135                 140

gct ctc atc aga aat aac atg caa cat ata atg ggg cca gtg act gcg       480
Ala Leu Ile Arg Asn Asn Met Gln His Ile Met Gly Pro Val Thr Ala
145                 150                 155                 160

att aaa ctc ctg ggg gaa gaa aac aaa gag aac aca cct agg aat gtt       528

Ile Lys Leu Leu Gly Glu Glu Asn Lys Glu Asn Thr Pro Arg Asn Val
                165                 170                 175

cta aac ata atc cca gca agt atg aat tat gct aaa gca cac tcg aag     576
Leu Asn Ile Ile Pro Ala Ser Met Asn Tyr Ala Lys Ala His Ser Lys
            180                 185                 190

gat aaa aag aag cct caa aga gat tcc caa gcc cag aaa agt cca gta     624
Asp Lys Lys Lys Pro Gln Arg Asp Ser Gln Ala Gln Lys Ser Pro Val
        195                 200                 205

aaa agc aaa agc acc cat cgt att caa cac aac att gac tac cta aaa     672
Lys Ser Lys Ser Thr His Arg Ile Gln His Asn Ile Asp Tyr Leu Lys
    210                 215                 220

cat ctc tca aaa gtc aaa aaa atc ccc agt gat ttt gaa ggc agc ggt     720
His Leu Ser Lys Val Lys Lys Ile Pro Ser Asp Phe Glu Gly Ser Gly
225                 230                 235                 240

tat aca gat ctt caa gag aga ggg gac aat gat ata tct cct ttc agt     768
Tyr Thr Asp Leu Gln Glu Arg Gly Asp Asn Asp Ile Ser Pro Phe Ser
                245                 250                 255

ggg gac ggc caa cct ttt aag gac att cct ggt aaa gga gaa gct act     816
Gly Asp Gly Gln Pro Phe Lys Asp Ile Pro Gly Lys Gly Glu Ala Thr
            260                 265                 270

ggt cct gac cta gaa ggc aaa gat att caa aca ggg ttt gca ggc cca     864
Gly Pro Asp Leu Glu Gly Lys Asp Ile Gln Thr Gly Phe Ala Gly Pro
        275                 280                 285

agt gaa gct gag agt act cat ctt gac aca aaa aag cca ggt tat aat     912
Ser Glu Ala Glu Ser Thr His Leu Asp Thr Lys Lys Pro Gly Tyr Asn
    290                 295                 300

gag atc cca gag aga gaa gaa aat ggt gga aat acc att gga act agg     960
Glu Ile Pro Glu Arg Glu Glu Asn Gly Gly Asn Thr Ile Gly Thr Arg
305                 310                 315                 320

gat gaa act gcg aaa gag gca gat gct gtt gat gtc agc ctt gta gag     1008
Asp Glu Thr Ala Lys Glu Ala Asp Ala Val Asp Val Ser Leu Val Glu
                325                 330                 335

ggc agc aac gat atc atg ggt agt acc aat ttt aag gag ctc cct gga     1056
Gly Ser Asn Asp Ile Met Gly Ser Thr Asn Phe Lys Glu Leu Pro Gly
            340                 345                 350

aga gaa gga aac aga gtg gat gct ggc agc caa aat gct cac caa ggg     1104
Arg Glu Gly Asn Arg Val Asp Ala Gly Ser Gln Asn Ala His Gln Gly
        355                 360                 365

aag gtt gag ttt cat tac cct cct gca ccc tca aaa gag aaa aga aaa     1152
Lys Val Glu Phe His Tyr Pro Pro Ala Pro Ser Lys Glu Lys Arg Lys
    370                 375                 380

gaa ggc agt agt gat gca gct gaa agt acc aac tat aat gaa att cct     1200
Glu Gly Ser Ser Asp Ala Ala Glu Ser Thr Asn Tyr Asn Glu Ile Pro
385                 390                 395                 400

aaa aat ggc aaa ggc agt acc aga aag ggt gta gat cat tct aat agg     1248
Lys Asn Gly Lys Gly Ser Thr Arg Lys Gly Val Asp His Ser Asn Arg
                405                 410                 415

aac caa gca acc tta aat gaa aaa caa agg ttt cct agt aag ggc aaa     1296
Asn Gln Ala Thr Leu Asn Glu Lys Gln Arg Phe Pro Ser Lys Gly Lys
            420                 425                 430

agt cag ggc ctg ccc att cct tct cgt ggt ctt gat aat gaa atc aaa     1344

Ser Gln Gly Leu Pro Ile Pro Ser Arg Gly Leu Asp Asn Glu Ile Lys
        435                 440                 445

aac gaa atg gat tcc ttt aat ggc ccc agt cat gag aat ata ata aca      1392
Asn Glu Met Asp Ser Phe Asn Gly Pro Ser His Glu Asn Ile Ile Thr
    450                 455                 460

cat ggc aga aaa tat cat tat gta ccc cac aga caa aat aat tct aca      1440
His Gly Arg Lys Tyr His Tyr Val Pro His Arg Gln Asn Asn Ser Thr
465                 470                 475                 480

cgg aat aag ggt atg cca caa ggg aaa ggc tcc tgg ggt aga caa ccc      1488
Arg Asn Lys Gly Met Pro Gln Gly Lys Gly Ser Trp Gly Arg Gln Pro
                485                 490                 495

cat tcc aac agg agg ttt agt tcc cgt aga agg gat gac agt agt gag      1536
His Ser Asn Arg Arg Phe Ser Ser Arg Arg Arg Asp Asp Ser Ser Glu
            500                 505                 510

tca tct gac agt ggc agt tca agt gag agc gat ggt gac                  1575
Ser Ser Asp Ser Gly Ser Ser Ser Glu Ser Asp Gly Asp
        515                 520                 525

<210>  2
<211>  525
<212>  PRT
<213>  Homo sapiens

<400>  2

Met Arg Val Phe Cys Val Gly Leu Leu Leu Phe Ser Val Thr Trp Ala
1               5                   10                  15

Ala Pro Thr Phe Gln Pro Gln Thr Glu Lys Thr Lys Gln Ser Cys Val
            20                  25                  30

Glu Glu Gln Arg Gln Glu Glu Lys Asn Lys Asp Asn Ile Gly Phe His
        35                  40                  45

His Leu Gly Lys Arg Ile Asn Gln Glu Leu Ser Ser Lys Glu Asn Ile
    50                  55                  60

Val Gln Glu Arg Lys Lys Asp Leu Ser Leu Ser Glu Ala Ser Glu Asn
65                  70                  75                  80

Lys Gly Ser Ser Lys Ser Gln Asn Tyr Phe Thr Asn Arg Gln Arg Leu
                85                  90                  95

Asn Lys Glu Tyr Ser Ile Ser Asn Lys Glu Asn Thr His Asn Gly Leu
            100                 105                 110

Arg Met Ser Ile Tyr Pro Lys Ser Thr Gly Asn Lys Gly Phe Glu Asp
        115                 120                 125

Gly Asp Asp Ala Ile Ser Lys Leu His Asp Gln Glu Glu Tyr Gly Ala
    130                 135                 140

Ala Leu Ile Arg Asn Asn Met Gln His Ile Met Gly Pro Val Thr Ala

145                 150                 155                 160

Ile Lys Leu Leu Gly Glu Glu Asn Lys Glu Asn Thr Pro Arg Asn Val
                165                 170                 175

Leu Asn Ile Ile Pro Ala Ser Met Asn Tyr Ala Lys Ala His Ser Lys
            180                 185                 190

Asp Lys Lys Lys Pro Gln Arg Asp Ser Gln Ala Gln Lys Ser Pro Val
        195                 200                 205

Lys Ser Lys Ser Thr His Arg Ile Gln His Asn Ile Asp Tyr Leu Lys
    210                 215                 220

His Leu Ser Lys Val Lys Lys Ile Pro Ser Asp Phe Glu Gly Ser Gly
225                 230                 235                 240

Tyr Thr Asp Leu Gln Glu Arg Gly Asp Asn Asp Ile Ser Pro Phe Ser
                245                 250                 255

Gly Asp Gly Gln Pro Phe Lys Asp Ile Pro Gly Lys Gly Glu Ala Thr
            260                 265                 270

Gly Pro Asp Leu Glu Gly Lys Asp Ile Gln Thr Gly Phe Ala Gly Pro
        275                 280                 285

Ser Glu Ala Glu Ser Thr His Leu Asp Thr Lys Lys Pro Gly Tyr Asn
    290                 295                 300

Glu Ile Pro Glu Arg Glu Glu Asn Gly Gly Asn Thr Ile Gly Thr Arg
305                 310                 315                 320

Asp Glu Thr Ala Lys Glu Ala Asp Ala Val Asp Val Ser Leu Val Glu
                325                 330                 335

Gly Ser Asn Asp Ile Met Gly Ser Thr Asn Phe Lys Glu Leu Pro Gly
            340                 345                 350

Arg Glu Gly Asn Arg Val Asp Ala Gly Ser Gln Asn Ala His Gln Gly
        355                 360                 365

Lys Val Glu Phe His Tyr Pro Pro Ala Pro Ser Lys Glu Lys Arg Lys
    370                 375                 380

Glu Gly Ser Ser Asp Ala Ala Glu Ser Thr Asn Tyr Asn Glu Ile Pro
385                 390                 395                 400

Lys Asn Gly Lys Gly Ser Thr Arg Lys Gly Val Asp His Ser Asn Arg
                405                 410                 415

Asn Gln Ala Thr Leu Asn Glu Lys Gln Arg Phe Pro Ser Lys Gly Lys

420                 425                 430

Ser Gln Gly Leu Pro Ile Pro Ser Arg Gly Leu Asp Asn Glu Ile Lys
        435                 440                 445

Asn Glu Met Asp Ser Phe Asn Gly Pro Ser His Glu Asn Ile Ile Thr
    450                 455                 460

His Gly Arg Lys Tyr His Tyr Val Pro His Arg Gln Asn Asn Ser Thr
465                 470                 475                 480

Arg Asn Lys Gly Met Pro Gln Gly Lys Gly Ser Trp Gly Arg Gln Pro
                485                 490                 495

His Ser Asn Arg Arg Phe Ser Ser Arg Arg Arg Asp Asp Ser Ser Glu
            500                 505                 510

Ser Ser Asp Ser Gly Ser Ser Ser Glu Ser Asp Gly Asp
        515                 520                 525

<210>  3
<211>  1662
<212>  DNA
<213>  Homo sapiens

<400>  3
ctcaaagatg cgagttttct gtgtgggact actccttttc agtgtgacct gggcagcacc      60

aacatttcaa ccacagactg agaaaactaa gcaaagctgt gtggaagagc agaggcagga     120

agaaaaaaac aaagacaata ttggttttca ccatttgggc aagagaataa atcaagagct     180

atcatctaaa gaaaatattg tccaggaaag aaagaaagat ttgtcccttt ctgaagccag     240

tgagaataag ggaagtagta aatctcaaaa ttatttcaca aatagacaga gactgaataa     300

agaatatagt atcagtaaca aagagaatac tcacaatggc ctgaggatgt caatttatcc     360

taagtcaact gggaataaag ggtttgagga tggagatgat gctatcagca aactacatga     420

ccaagaagaa tatggcgcag ctctcatcag aaataacatg caacatataa tggggccagt     480

gactgcgatt aaactcctgg gggaagaaaa caaagagaac acacctagga atgttctaaa     540

cataatccca gcaagtatga attatgctaa agcacactcg aaggataaaa agaagcctca     600

aagagattcc caagcccaga aaagtccagt aaaaagcaaa agcacccatc gtattcaaca     660

caacattgac tacctaaaac atctctcaaa agtcaaaaaa atccccagtg attttgaagg     720

cagcggttat acagatcttc aagagagagg ggacaatgat atatctcctt tcagtgggga     780

cggccaacct tttaaggaca ttcctggtaa aggagaagct actggtcctg acctagaagg     840

caaagatatt caaacagggt ttgcaggccc aagtgaagct gagagtactc atcttgacac     900

aaaaaagcca ggttataatg agatcccaga gagagaagaa aatggtggaa ataccattgg     960

aactagggat gaaactgcga aagaggcaga tgctgttgat gtcagccttg tagagggcag    1020

caacgatatc atgggtagta ccaattttaa ggagctccct ggaagagaag gaaacagagt    1080

ggatgctggc agccaaaatg ctcaccaagg gaaggttgag tttcattacc ctcctgcacc 1140

ctcaaaagag aaaagaaaag aaggcagtag tgatgcagct gaaagtacca actataatga 1200

aattcctaaa aatggcaaag gcagtaccag aaagggtgta gatcattcta ataggaacca 1260

agcaacctta aatgaaaaac aaaggtttcc tagtaagggc aaaagtcagg gcctgcccat 1320

tccttctcgt ggtcttgata atgaaatcaa aaacgaaatg gattccttta atggccccag 1380

tcatgagaat ataataacac atggcagaaa atatcattat gtaccccaca gacaaaataa 1440

ttctacacgg aataagggta tgccacaagg gaaaggctcc tggggtagac aaccccattc 1500

caacaggagg tttagttccc gtagaaggga tgacagtagt gagtcatctg acagtggcag 1560

ttcaagtgag agcgatggtg actagtccac caggagttcc cagcggggtg acagtctgaa 1620

gacctcgtca cctgtgagtt gatgtagagg agagccacct ga 1662

<210> 4
<211> 66
<212> DNA
<213> Artificial

<220>
<223> Primer

<400> 4
gagagagaga gagagagaga acgcgtcgac tcgagcggcc gcggaccgtt tttttttttt 60

tttttt 66

<210> 5
<211> 19
<212> DNA
<213> Artificial

<220>
<223> Primer

<400> 5
ggaaacagct atgaccatg 19

<210> 6
<211> 30
<212> DNA
<213> Artificial

<220>
<223> Primer

<400> 6
tcaggtggct ctcctctaca tcaactcaca 30

<210> 7
<211> 28
<212> DNA
<213> Artificial

<220>
<223> Primer

<400>  7
ctcaaagatg cgagttttct gtgtggga                                          28

## Description

### Technical Field

The present invention relates to a plural number of novel antibodies having a different antigenic determinant against Matrix extracellular phosphoglycoprotein (hereinafter to be abbreviated as "MEPE") and use thereof, specifically, use as a diagnostic agent for osteoporosis.

### Background Art

MEPE is highly expressed in causative tumors of oncogenic hypophosphatemic osteomalacia (hereinafter to be sometimes abbreviated as "OHO") patients, is a glycoprotein genetically cloned therefrom (in human, consisting of 525 amino acids in full-length; SEQ ID NO: 2), and is thought to be a candidate for a phosphaturic factor (Phosphatonin), which causes hypophosphatemia. Researches in recent years have revealed that (1) MEPE is strongly expressed in bone tissues (A. Nampei et al., J. Bone Miner. Metab., 22: 176-84 (2004)), (2) the expression thereof increases as the differentiation/calcification of osteoblast progresses, (3) the bone mass increases in a MEPE knockout mouse, and the differentiation of osteoblast is enhanced (L.C. Gowen et al., J. Biol. Chem., 278: 1998-2007 (2003)), and the like; hence, it has been suggested that MEPE functions as a bone morphogenetic inhibitory factor. However, it has been suggested recently that a fragment of MEPE exhibit an action different from that of the full-length protein.

To clarify the correlation of MEPE with pathology, measuring the concentration of MEPE or MEPE decomposition products in the blood is important; the possibility that the level of MEPE in blood be useful as a pathological marker of bone/joint diseases is anticipated. Thus far, A. Jain et al. (J. Clin. Endocrinol. Metab., 89: 4158-61 (2004)) measured the MEPE concentration in blood of normal individuals by ELISA using an anti-MEPE polyclonal antibody, and reported that there was positive correlation between MEPE and serum phosphoric acid value, PTH value, femur BMD, and total hip BMD.

### Disclosure of the Invention

It is an object of the present invention to provide various novel anti-MEPE antibodies for detecting a MEPE-derived molecule in blood, and to provide a method of detecting a MEPE-derived molecule that can be a pathological marker of bone/joint diseases in blood using the antibodies.

To achieve the above-mentioned objects, the present inventors prepared 8 kinds of anti-human MEPE mouse monoclonal antibodies and rabbit polyclonal antibodies, and constructed a plural number of sandwich ELISA systems with varying combinations of the antibodies. The MEPE concentration in plasma collected from postmenopausal females were measured using these assay systems to find that the measurement results were sometimes remarkably different depending on the combination of the antibodies. Of these, when the two kinds of monoclonal antibodies, MAB-ME-16F4.3 and MAB-ME-12C9.2, were used, the results were of the detection limit or below in about 70 percent of the plasmas examined. Furthermore, unexpectedly, it was clarified that plasma donors in whose plasma MEPE was not more than detection limit in the assay systems have significantly high frequency of bone fracture compared to plasma donors in whose plasma MEPE was detected. Hence, the present inventors investigated regions in MEPE recognized by each of the monoclonal antibodies MAB-ME-16F4.3 and MAB-ME-12C9.2, and elucidated that diagnosis of osteoporosis and risk prediction of bone fracture became possible by checking whether or not a MEPE-derived molecule containing both of the regions was present in plasma.

The present inventors further studied based on these findings, and resulted in the completion of the present invention.

Accordingly, the present invention provides
[1] an antibody of the following (a) or (b):
   (a) mouse monoclonal antibody MAB-ME-16F4.3 (FERM BP-10329)
   (b) an antibody capable of specifically recognizing the same amino acid sequence as that of an antigenic determinant of the antibody of the above-mentioned (a);
[2] the antibody described in the above-mentioned [1], wherein the antigenic determinant is present in the amino acid sequence shown by amino acid numbers 253-261 in the amino acid sequence shown by SEQ ID NO: 2;
[3] an antibody of the following (a) or (b);
   (a) mouse monoclonal antibody MAB-ME-12C9.2 (FERM BP-10328)
   (b) an antibody capable of specifically recognizing the same amino acid sequence as that of an antigenic determinant of the antibody of the above-mentioned (a);
[4] the antibody described in the above-mentioned [3], wherein the antigenic determinant is present in the amino acid sequence shown by amino acid numbers 298-306 in the amino acid sequence shown by SEQ ID NO: 2;
[5] a method for predicting the risk of bone fracture and/or diagnosis of osteoporosis, which comprises using the antibody described in the above-mentioned [1] and the antibody described in the above-mentioned [3];
[6] the method described in the above-mentioned [5], which comprises measuring a substance in a biological sample, wherein the substance is recognized by the antibody described in the above-mentioned [1] and the antibody described in the above-mentioned [3];
[7] the method described in the above-mentioned [6], which comprises measuring the substance by sandwich method;
[8] the method described in the above-mentioned [6], wherein the biological sample is blood, serum or plasma; and
[9] a kit for predicting the risk of bone fracture and/or diagnosis of osteoporosis, comprising the antibody described in the above-mentioned [1] and the antibody described in the above-mentioned [3]; and the like.

The two kinds of the anti-MEPE antibodies of the present invention afford the advantageous effect that by measuring MEPE-derived molecules in a biological sample using the antibodies, it is possible to predict the risk of bone fracture of the animal from which the sample has been collected, and to diagnose osteoporosis.

### Brief Description of the Drawings

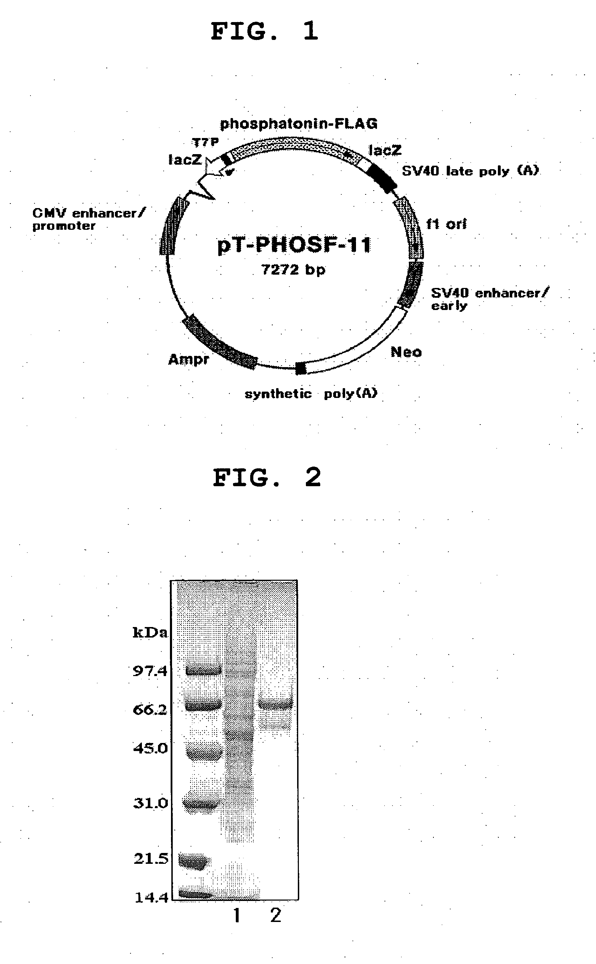

Fig. 1 shows the structure of the animal cell expression plasmid for phosphatonin (MEPE)-FLAG adduct. In the figure, Phosphatonin-FLAG indicates the coding sequence of the phosphatonin (MEPE)-FLAG adduct, T7P indicates T7 promoter, f1 ori indicates f1 replication origin, Neo indicates neomycin resistance gene, and Ampr indicates ampicillin resistant gene.
Fig. 2 shows the SDS-polyacrylamide gel electrophoresis profile of MEPE-FLAG for insect cell-expression obtained in Example 3. The migration was performed using Multi gel 4/20 under reducing condition, treated with 100 mM DTT at 100°C for 2 min.
   Lane 1: insect cell culture supernatant; Lane 2: purified MEPE-FLAG preparation (1 µg)

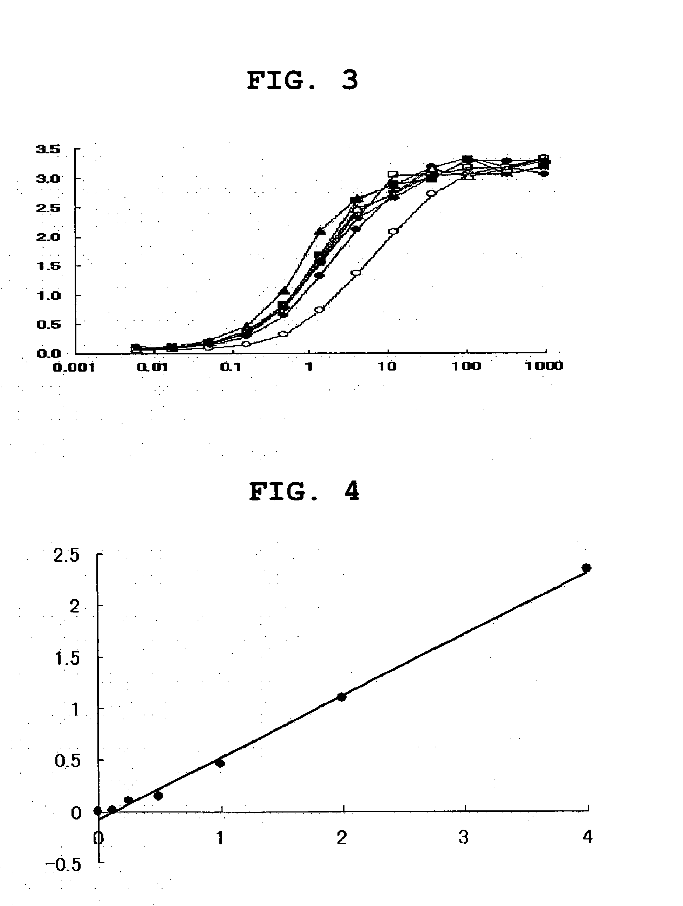

Fig. 3 shows the binding affinity of 8 kinds of the anti-MEPE mouse monoclonal antibodies described in Example 4 with insolubilized MEPE-FLAG. The vertical axis indicates the absorbance at 450 nm, and the horizontal axis does the concentration of antibody (ng/ml), respectively.
   -●-: ME-2D11.9; -▲-: ME-4A2.2; -■-: ME-9D2.15; -○-: ME-9H6.8; -*-; ME-10E4.2; -Δ-: ME-12C9.2; -□-: ME-12E2.1; -**◆**-: ME-16F4.3
Fig. 4 shows the calibration curve of the enzyme immunoassay method for measuring the concentration of MEPE-derived antigen in plasma described in Example 5. The vertical axis indicates the absorbance at 450 nm, and the horizontal axis does the concentration of the antigen (ng/ml), respectively.

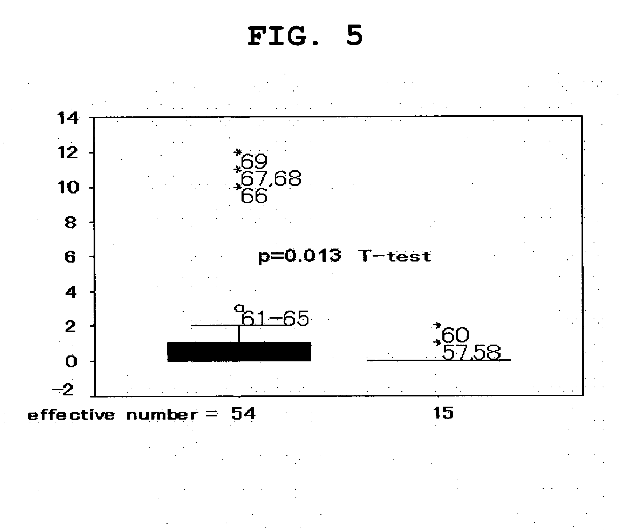

Fig. 5 shows the correlation between the concentration of MEPE-derived antigen in plasma from postmenopausal females without administration of bisphosphonate, and the number of fracture of vertebra. The vertical axis indicates the number of fracture of vertebra, while the horizontal axis does the case in which the concentration of MEPE-derived antigen in plasma is not more than detection limit (<0.59 ng/ml; effective number: 54 samples) or is not less than detection limit (<0.59 ng/ml; effective number: 15 samples), from left. The symbols (* and o) in the graph indicate plots of samples protruding from the error bars, and the sample number is written in addition at the lower right of each of the plots.

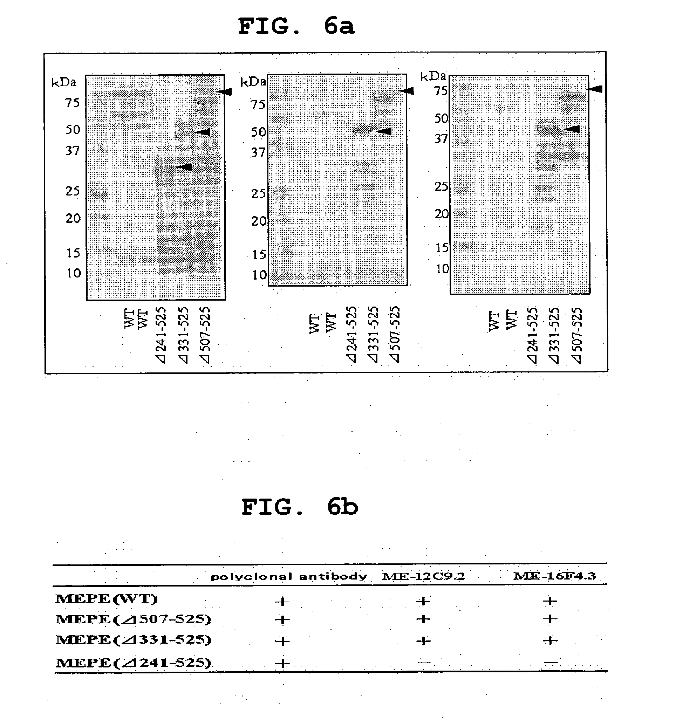

Fig. 6 shows the binding specificity (Western blot) of an anti-MEPE rabbit polyclonal antibody (Fig. 6A, the left panel) and 2 kinds of anti-MEPE mouse monoclonal antibodies obtained in Example 4 (MAB-ME-12C9.2 (Fig. 6A, the middle panel), MAB-ME-16F4.3 (Fig. 6A, the right panel)) with various MEPE C-terminal deletion muteins. In the figure, WT indicates wild-type MEPE-FLAG (mature-type full-length MEPE +FLAG tag), and Δ241-525, Δ331-525 and Δ507-525 indicate C-terminal deletion muteins, wherein positions-241-525, 331-525 or 507-525 of mature-type MEPE-FLAG are deleted, respectively. In Fig. 6B, "+" indicates that the antibody has binding activity, while "-" indicates that the antibody doesn't have binding activity, respectively.

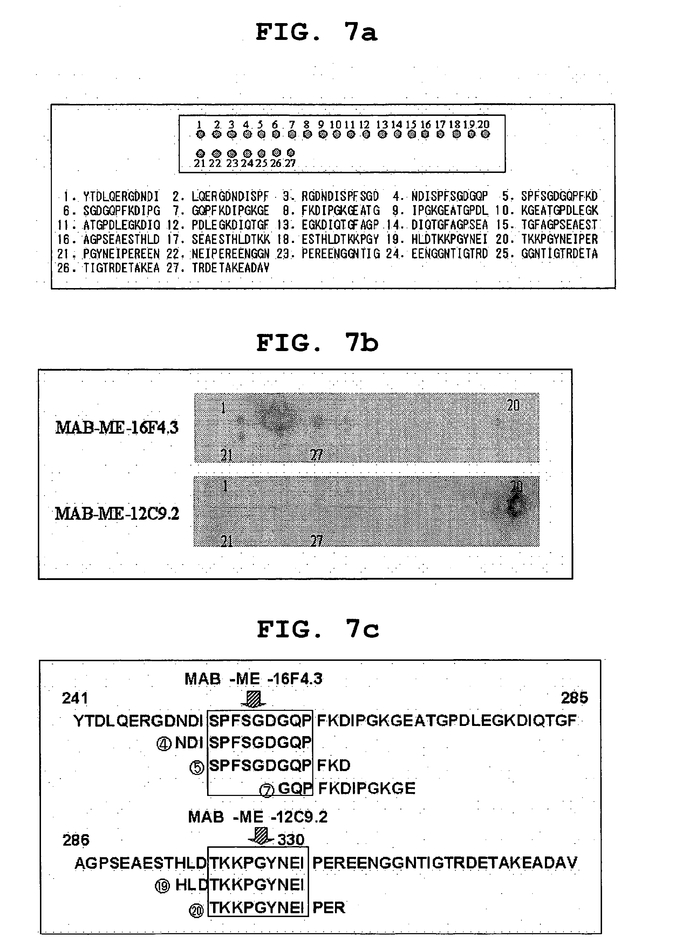

Fig. 7 shows the binding specificity of 2 kinds of anti-MEPE mouse monoclonal antibodies obtained in Example 4 (MAB-ME-12C9.2, MAB-ME-16F4.3) with synthesized partial peptides of MEPE. Fig. 7A shows the arrangement of spots in immunodot blotting, and the amino acid sequences of synthesized partial peptides of MEPE that are solid-phase synthesized at the position of each spot number. Fig. 7B shows the results obtained from the immunodot blotting (the upper panel: MAB-ME-12C9.2; the lower panel: MAB-ME-16F4.3). Numbers in the figure indicates spot numbers. Fig. 7C shows the alignment between the amino acid sequence of synthesized partial peptides of MEPE to which each of the antibodies (the upper panel: MAB-ME-12C9.2; the lower panel: MAB-ME-16F4.3) demonstrated binding activity and the corresponding amino acid sequence of MEPE. For each, the sequence in the most upper line indicates a partial amino acid sequence of MEPE, and the numbers appended above the sequence indicates amino acid numbers. The number with circle indicates a spot number. From the results of the alignment, it was determined that antigenic determinants of each of the antibodies were present in the partial sequences enclosed by the box.

### Best Mode for Embodying the Invention

The first anti-MEPE antibody of the present invention (hereinafter to be sometimes abbreviated as "the first antibody of the present invention") is anti-human MEPE mouse monoclonal antibody MAB-ME-16F4.3 (hereinafter, to be sometimes simply abbreviated as "MAB-ME-16F4.3"), or antibodies capable of specifically recognizing the same amino acid sequence as an antigenic determinant of the antibody (epitope 1; E1). While the antibody may be a monoclonal antibody or a polyclonal antibody, as long as being capable of specifically recognizing E1, it is preferable to be a monoclonal antibody. Although the isotype of the antibody is not subject to limitation, preferably IgG, IgM or IgA, particularly preferably IgG, can be mentioned. Also, the immunogen and the immunized animal are not particularly limited; for example, the first antibody of the present invention can include antibodies obtained by using a region in a MEPE ortholog corresponding to E1, which is derived, for example, from a non-human mammal (e.g., monkey, chimpanzee, swine, bovine, horse, sheep, cat, dog, mouse, rat, rabbit and the like) (E1'; for example, in the case of cynomolgus monkey, E1' is present in the amino acid sequence shown by amino acid numbers 284-292 in the amino acid sequence registered in GenBank as Accession Number BAB17010.1) as an immunogen, antibodies obtained by immunizing MEPE or a fragment thereof to a non-human warm-blooded animal (e.g., rabbit, goat, bovine, chick, mouse, rat, sheep, swine, horse, cat, dog, monkey, chimpanzee and the like), a human culture cell or the like, antibodies obtained by the antibody display method, such as a phage display method, using an antibody gene library of a non-human warm-blooded animal (e.g., rabbit, goat, bovine, chick, mouse, rat, sheep, swine, horse, cat, dog, monkey, chimpanzee and the like) or human, and the like.

The antigenic determinant of MAB-ME-16F4.3 (E1) is present in the amino acid sequence shown by amino acid numbers 253-261 in the amino acid sequence shown by SEQ ID NO: 2. Whether or not a given anti-MEPE antibody is capable of recognizing the same amino acid sequence as E1 is examined, for example, by checking the inhibition of binding of MAB-ME-16F4.3 with MEPE using a general cross-blocking assay such as one described in Antibodies, A Laboratory Manual, Cold Spring Harbor Laboratory, Harlow and David Lane ed. (1998). Alternatively, it can be more directly examined by checking whether or not the antibody binds with a peptide containing E1 as in MAB-ME-16F4.3 by epitope mapping using peptide array, phage display or the like. MAB-ME-16F4.3 was deposited at the International Patent Organism Depositary (IPOD), National Institute of Advanced Industrial Science and Technology (AIST Tsukuba Central 6, 1-1-1 Tsukuba West, Tsukuba, Ibaraki 305-8566, Japan), as deposit number FERM BP-10329 on April 26, 2005.

The second anti-MEPE antibody of the present invention (hereinafter to be sometimes abbreviated as "the second antibody of the present invention") is anti-human MEPE mouse monoclonal antibody MAB-ME-12C9.2 (hereinafter, to be sometimes simply abbreviated as "MAB-ME-12C9.2"), or antibodies capable of recognizing the same amino acid sequence as an antigenic determinant of the antibody (epitope 2; E2). While the antibody may be a monoclonal antibody or a polyclonal antibody, as long as being capable of specifically recognizing E2, it is preferable to be a monoclonal antibody. Although the isotype of the antibody is not subject to limitation, preferably IgG, IgM or IgA, particularly preferably IgG, can be mentioned. Also, the immunogen and the immunized animal are not particularly limited; for example, the second antibody of the present invention can include antibodies obtained by using a region in a MEPE ortholog corresponding to E2, which is derived, for example, from a non-human mammal (e.g., monkey, chimpanzee, swine, bovine, horse, sheep, cat, dog, mouse, rat, rabbit and the like) (E2'; for example, in the case of crab-eating monkey, E2' is present in the amino acid sequence shown by amino acid numbers 329-337 in the amino acid sequence registered in GenBank as Accession Number BAB17010.1) as an immunogen, antibodies obtained by immunizing MEPE or a fragment thereof to a non-human warm-blooded animal (e.g., rabbit, goat, bovine, chick, mouse, rat, sheep, swine, horse, cat, dog, monkey, chimpanzee and the like), a human culture cell or the like, antibodies obtained by the antibody display method, such as a phage display method, using an antibody gene library of a non-human warm-blooded animal (e.g., rabbit, goat, bovine, chick, mouse, rat, sheep, swine, horse, cat, dog, monkey, chimpanzee and the like) or human, and the like.

The antigenic determinant of MAB-ME-12C9.2 (E2) is present in the amino acid sequence shown by amino acid numbers 298-306 in the amino acid sequence shown by SEQ ID NO: 2. Whether or not a given anti-MEPE antibody is capable of recognizing the same amino acid sequence as E2 is examined, for example, by checking the inhibition of binding of MAB-ME-12C9.2 with MEPE using a general cross-blocking assay such as one described in Antibodies, A Laboratory Manual, Cold Spring Harbor Laboratory, Harlow and David Lane ed. (1998). Alternatively, it can be more directly examined by checking whether or not the antibody binds with a peptide containing E2 as in MAB-ME-12C9.2 by epitope mapping using peptide array, phage display or the like. MAB-ME-12C9.2 was deposited at the International Patent Organism Depositary (IPOD), National Institute of Advanced Industrial Science and Technology (AIST Tsukuba Central 6, 1-1-1 Tsukuba West, Tsukuba, Ibaraki 305-8566, Japan), as deposit number FERM BP-10328 on April 26, 2005.

The molecular form of the first and the second antibody of the present invention is not subject to limitation, as long as they have at least a complementarity determining region (CDR) for specifically recognizing and binding to each of the antigenic determinants; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like, and the like.

When the antibody of the present invention is a monoclonal antibody, it can be prepared by, for example, the following method.

### (1) Preparation of immunogen

As the immunogen used, human MEPE or a fragment thereof containing E1 (or E2), other mammal-derived MEPE ortholog or a fragment thereof containing the region corresponding to E1 (or E2) (E1' (or E2')), a synthetic peptide containing E1 (or E2) or E1' (or E2'), and the like can be mentioned.

In the present specification, regarding the peptides and proteins shown by an amino acid sequence, the left end is the N-terminal (amino terminal) and the right end is the C terminal (carboxyl terminal) in accordance with the conventional peptide marking. The C-terminal of MEPE in the present invention or the fragment thereof, or the synthetic peptide containing the amino acid sequence of the antigenic determinant recognized by the antibody of the present invention (hereinafter to be referred comprehensively as "antigen peptide") can be any of carboxyl group, carboxylate, amide or ester. Additionally, when the antigen peptide has a carboxyl group (or carboxylate) at other than the C-terminal thereof, the carboxyl group can be amidated or esterified. Furthermore, the antigen peptide can be one whose amino group of the N-terminal amino acid residue is substituted, for example, with formyl group, acetyl group or the like, one whose N-terminal glutamine residue is pyroglutamated, or one whose substituent at an amino acid side chain in the molecule (e.g., -OH, -SH, amino group, imidazole group, indol group, guanidino group and the like) is substituted with other substituent (e.g., formyl group, acetyl group and the like).

The antigen peptide can be a salt with an acid or a base, and an acid addition salt is particularly preferable. Useful salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The antigen peptide can be obtained by (a) isolating and purifying from an antigen-producing tissue or cells of a mammal, for example, a human, monkey, rat, mouse and the like, by using a method known *per se* or a method based thereon, (b) chemically synthesizing by a method of peptide synthesis known *per se,* using a peptide synthesizer and the like (c) culturing a transformant comprising a DNA that encodes the antigen peptide, or by (d) biochemically synthesizing by using a cell-free transcription/translation system with a nucleic acid that encodes the antigen peptide as a template.
(a) When the antigen is prepared from a mammalian tissue or cells, it is possible to isolate and purify the antigen by homogenizing the tissue or cells, thereafter performing extraction with an acid or alcohol and the like, and subjecting the extract to a protein separation technique known *per se* (e.g., salting out, dialysis, chromatographies such as gel filtration chromatography, reversed phase chromatography, ion-exchange chromatography, and affinity chromatography, and the like). The antigen peptide obtained can be used as the immunogen as is, and can also be used as the immunogen in the form of a partial peptide prepared by limited degradation using a peptidase and the like.
(b) When the antigen peptide is chemically synthesized, the synthetic peptide is exemplified by ones having the same structure as the above-described antigen peptide purified from naturally occurring substances; to be specific, in the amino acid sequence of the natural antigen peptide, a peptide containing the same amino acid sequence as the amino acid sequence of E1 (or E1') or E2 (or E2'), which are the antigenic determinant of the first or the second antibody of the present invention, is used.
(c) When the antigen peptide is produced using a transformant comprising a DNA, the DNA can be prepared according to a known cloning method [for example, a method described in Molecular Cloning, 2nd ed.; J. Sambrook et al., Cold Spring Harbor Lab. Press (1989), and the like]. As the cloning method, for example, a method for (i) isolating a DNA that encodes the antigen from a cDNA library by the hybridization method using DNA probes designed on the basis of the gene sequence that encodes the antigen peptide (for example, in the case of human MEPE, as the sequence containing a base sequence encoding E1, the base sequence shown by base numbers 757-783 in the base sequence shown by SEQ ID NO: 1, and as the sequence containing a base sequence that encodes E2, the base sequence shown by base numbers 892-918 in the base sequence shown by SEQ ID NO: 1, respectively, can be mentioned), (ii) preparing a DNA that encodes the antigen peptide by a PCR method using DNA primers designed on the basis of the gene sequence that encodes the antigen peptide with a cDNA as the template, and inserting the DNA into an expression vector matching a host, thereafter transforming the host with the expression vector, and culturing the thus-obtained transformant in a suitable medium, and the like can be mentioned.
(d) When a cell-free transcription/translation system is utilized, a method for synthesizing an mRNA by using an expression vector incorporating a DNA that encodes the antigen peptide (e.g., an expression vector wherein the DNA is placed under the control of the T7 or SP6 promoter and the like, and the like) as the template, that was prepared by the same method as (c) above, a transcription reaction mixture comprising an RNA polymerase matching the promoter, and its substrates (NTPs); and thereafter performing a translation reaction with the mRNA as the template using a known cell-free translation system (e.g., *E*. *coli,* rabbit reticulocytes, extract from wheat germ etc.), and the like can be mentioned. By adjusting the salt concentration and the like appropriately, the transcription reaction and the translation reaction can also be carried out in the same reaction mixture at one time.

In the case of using a polypeptide such as MEPE full-length protein (e.g., not less than 21 amino acid residues) as the immunogen, the selection can be performed by cross-blocking assay or epitope mapping with the binding ability with E1 or E2 being the index.

Meanwhile, in the case of using an oligopeptide of not more than 20 amino acids as the immunogen, for example, an oligopeptide consisting of not less than 3, preferably not less than 4, more preferably not less than 5, and still more preferably not less than 6, continuous amino acid residues containing the amino acid sequence of E1 (or E1') or E2 (or E2') can be mentioned. Alternatively, as the oligopeptide, for example, an oligopeptide consisting of not more than 20, preferably not more than 18, more preferably not more than 15, and still more preferably not more than 12, continuous amino acid residues containing the amino acid sequence of E1 (or E1') or E2 (or E2') can be mentioned.

Although such the oligopeptides can be produced according to the methods of the above-mentioned (b) to (d), or by cleaving the antigen peptide prepared by the method of the above-mentioned (a) to (d) with a suitable peptidase or the like, it is preferable to use a known method of peptide synthesis.

The method of peptide synthesis may, for example, be any of solid phase synthesis and liquid phase synthesis. Specifically, the desired peptide can be produced by condensing a partial peptide or amino acids that can constitute the peptide and the remaining portion, and removing the protecting group when the product has a protecting group. Condensation and removal of protecting group can be achieved by known methods, for example, the methods described in (1) or (2) below.
1) M. Bodanszky and M.A. Ondetti, Peptide Synthesis, Interscience Publishers, New York (1966)
2) Schroeder and Luebke, The Peptide, Academic Press, New York (1965)

After the reaction, the peptide can be purified and isolated using conventional methods of purification, such as solvent extraction, distillation, column chromatography, liquid chromatography, recrystallization, etc., in combination thereof. When the peptide obtained by the above-mentioned method is in a free form, it can be converted to a suitable salt by a known method; conversely, when the peptide is obtained in the form of a salt, the salt can be converted to a free form or other salt by a known method.

For an amide form of peptide, commercially available resins for protein synthesis, which are suitable for amide formation, can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such resins, amino acids having α-amino groups and side-chain functional groups appropriately protected are condensed on the resin in accordance with the sequence of the desired peptide according to various condensation methods known *per se.* At the end of the reaction, the peptide is excised from the resin and the various protecting groups are removed simultaneously, thus affording the desired peptide. Alternatively, the desired peptide can also be obtained by using chlorotrityl resin, oxime resin, 4-hydroxybenzoic acid resin and the like, excising a partially protected peptide, and further removing protecting groups by a conventional method.

For the above-described condensation of protected amino acids, various activation reagents for peptide synthesis may be used, with preference given to carbodiimides. As the carbodiimides, DCC, N,N'-diisopropylcarbodiimide, N-ethyl-N'-(3-dimethylaminopropyl)carbodiimide and the like can be mentioned. For activation with these reagents, protected amino acids may be added directly to the resin along with a racemization inhibitor (e.g., HOBt, HOOBt etc.), or may be added to the resin after being previously activated to the form of a symmetric acid anhydride, HOBt ester, or HOOBt ester. Solvents to be used in the activation of protected amino acids or condensation with the resin can be selected as appropriate from among the solvents known to be usable for peptide condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; tertiary amines such as pyridine; ethers such as dioxane and tetrahydrofuran; nitriles such as acetonitrile and propionitrile; esters such as methyl acetate and ethyl acetate; appropriate mixtures of these solvents, and the like can be used. Reaction temperature is selected as appropriate from the range known to be useful for peptide bond formation reactions, and is normally selected as appropriate from the range of about -20°C to about 50°C. The activated amino acid derivative is normally used in an excess of about 1.5 to about 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole so that an influence on the subsequent reactions can be avoided.

Protection and protecting groups for the functional groups that should not involve the reaction of the starting amino acid, splitting off the protecting groups, activation of the functional groups involved in the reaction, and the like can be selected as appropriate from among known groups or known means.

Examples of the protecting groups for the amino groups of the starting amino acid include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like. Examples of the protecting group for the carboxyl groups include, for example, C₁₋₆alkyl group (e.g., methyl, ethyl, n-propyl, isopropyl, n-butyl), C₃₋₈cycloalkyl group (e.g., cyclopentyl, cyclohexyl), C₇₋₁₄aralkyl group (e.g., benzyl, phenethyl, naphthylmethyl), 2-adamantyl, 4-nitrobenzyl, 4-methoxybenzyl, 4-chlorobenzyl, phenacyl and benzyloxycarbonyl hydrazide, tertiary butoxycarbonyl hydrazide, trityl hydrazide and the like.

The hydroxyl group of serine or threonine can be protected by, for example, esterification or etherification. Examples of groups suitable for this esterification include lower (C₁₋₆) alkanoyl groups such as acetyl group, aroyl groups such as benzoyl group, groups derived from carbonic acid, such as benzyloxycarbonyl group and ethoxycarbonyl group, and the like. As examples of groups suitable for the etherification, benzyl group, tetrahydropyranyl group, t-butyl group and the like can be mentioned.

Examples of the protecting group for the phenolic hydroxyl group of tyrosine include Bzl, Cl-Bzl, 2-nitrobenzyl, Br-Z, tertiary butyl and the like.

Examples of the protecting group for the imidazole moiety of histidine include Tos, 4-methoxy-2,3,6-trimethylbenzenesulfonyl, DNP, Bom, Bum, Boc, Trt, Fmoc and the like.

Examples of activated carboxyl groups in the starting material include corresponding acid anhydrides, azides, activated esters (esters with alcohols (e.g., pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)]. Examples of activated amino groups in the starting material include corresponding phosphoric amides.

As examples of the method used to remove (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting of the protecting group by the above-described acid treatment is normally performed at a temperature of about -20°C to 40°C; in the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole moiety of histidine is removed by thiophenol treatment; the formyl group used as the protecting group for the indole moiety of tryptophan is removed by alkali treatment with dilute sodium hydroxide solution, dilute ammonia or the like, as well as by the above-described acid treatment in the presence of 1,2-ethanedithiol, 1,4-butanedithiol or the like.

In another method of obtaining an amide of the peptide, for example, the α-carboxyl group of the carboxy-terminal amino acid is first protected by amidation, and the peptide chain on the amino group side is then extended to a desired length; thereafter, a peptide having only the protecting group for the N-terminal α-amino group in the peptide chain removed and a peptide having only the protecting group for the C-terminal carboxyl group removed are prepared, and the two peptides are condensed in a mixed solvent as described above. Details of the condensation reaction are the same as those described above. After the protected peptide obtained by the condensation is purified, all the protecting groups are removed by the above-described method to give the desired crude peptide. This crude peptide may be purified by various known means of purification, and the major fraction may be lyophilized to give an amide of the desired peptide.

An ester of the peptide can be obtained by, for example, condensing the α-carboxyl group of the carboxy-terminal amino acid with a desired alcohol to prepare an amino acid ester, and then following the same procedures as those for the amide of the peptide.

The antigen peptide permit direct use for immunization in an insolubilized form, as long as it has immunogenicity; when an antigen of low molecular weight (e.g., molecular weight about 3,000 or less) having only one to several antigenic determinants in the molecule thereof (e.g., the above-described oligo peptide and the like) is used, it can be used for immunization in the form of a complex bound or adsorbed to a suitable carrier because these antigen peptides are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken, hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned. Regarding the mixing ratio of the carrier and hapten, any combination in any ratio can be bound or adsorbed, as long as an antibody against the antigen bound or adsorbed to the carrier is produced efficiently; usually, one wherein the above-described naturally occurring or synthetic polymer carrier in common use in preparing an antibody against hapten is bound or adsorbed in a ratio by weight of 0.1 to 100 to 1 of hapten can be used.

Various condensing agents can be used for coupling the hapten and carrier protein. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like can be used advantageously. When amino groups are crosslinked together, it is also possible to react one amino group with an activated ester reagent having a dithiopyridyl group (e.g., SPDP and the like), followed by reduction, to introduce the thiol group, and to introduce a maleimide group into the other amino group using a maleimide activated ester reagent, followed by a reaction of both. Also, it is possible to add a cysteine residue to the N-terminal or C-terminal of the hapten (peptide), and to introduce a maleimide group into the amino group using a maleimide activated ester reagent, followed by a reaction of both.

### (2) Preparation of monoclonal antibody

An antigen peptide is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about two to 10 times in total every 1 to 6 weeks. As examples of the warm-blooded animal used, rabbit, goat, bovine, chicken, mouse, rat, hamster, sheep, swine, horse, camel, cat, dog, monkey, chimpanzee and the like can be mentioned, and mice and rat are generally preferably used for generating a monoclonal antibody.

As to preparing a monoclonal antibody-producing cell, a monoclonal antibody-producing hybridoma can be prepared by selecting an individual in which serum antibody titer has been observed from a mammal (e.g., mouse) immunized with the antigen, isolating a spleen or a lymph node 2 to 5 days after the final immunization, and fusing an antibody-producing cell contained therein with a myeloma cell. The measurement of the antibody titer in an antiserum can be performed, for example, by reacting an insolubilized antigen peptide with the antiserum, followed by detecting an antigen peptide-specific antibody binding to the solid phase using an antibody labeled with a radioactive substance or an enzyme against the antibody of the animal species immunized. The fusion procedure can be performed according to a known method, for example, the method of Köhler and Milstein [Nature, 256, 495 (1975)]. As examples of a fusogen, polyethylene glycol (PEG), Sendai virus and the like can be mentioned, and PEG is preferably used.

As examples of the myeloma cell, NS-1, P3U1 and SP2/O can be mentioned, and P3U1 is preferably used. A preferable ratio of the number of antibody-producing cells (splenocytes) and number of myeloma cells used is about 1:1 to 20:1; cell fusion can be efficiently performed by adding a PEG (preferably PEG1000 to PEG6000) at concentrations of about 10 to 80%, and conducting incubation at about 20 to 40°C, preferably at about 30 to 37°C, for about 1 to 10 minutes.

Selection of the fusion cell (hybridoma) can be conducted according to a method known *per se* or a method based thereon. Selection thereof can normally be conducted using an animal cell culture medium supplemented with HAT (hypoxanthine, aminopterin, thymidine). As the medium for selection and breeding thereof, any medium can be used, as long as the hybridoma can grow therein. For example, an RPMI 1640 medium containing 1 to 20%, preferably 10 to 20%, fetal bovine serum, a GIT medium (Wako Pure Chemical Industries, Ltd.) containing 1 to 10% fetal bovine serum or a serum-free medium for hybridoma culture (SFM-101, Nissui Pharmaceutical Co., Ltd.) and the like can be used. Cultivation temperature is normally 20 to 40°C, preferably about 37°C. Cultivation time is normally 5 days to 3 weeks, preferably 1 week to 2 weeks. Cultivation can normally be conducted under 5% carbonic acid gas.

For screening the monoclonal antibody-producing hybridoma, various methods can be used; examples can include the method wherein a hybridoma culture supernatant is added to a solid phase (e.g. microplate) on which the antigen peptide is adsorbed directly or with a carrier, subsequently an anti-immunoglobulin antibody labeled with a radioactive substance, an enzyme or the like (when the antibody-producing cell used for cell fusion is from mouse, an anti-mouse immunoglobulin antibody is used) or protein A is added thereto, and a monoclonal antibody binding to the solid phase is detected; the method wherein a hybridoma culture supernatant is added to a solid phase on which an anti-immunoglobulin antibody or protein A is adsorbed, the antigen peptide labeled with a radioactive substance or an enzyme or the like, etc. are added thereto, and a monoclonal antibody binding to the solid phase is detected, and the like.

### (3) Purification of the monoclonal antibody

Separation and purification of the monoclonal antibody are performed according to a method of immunoglobulin separation and purification [e.g., salting-out, alcohol precipitation, isoelectric point precipitation, electrophoresis, adsorption-desorption with an ion exchanger (e.g., DEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like] in the same manner as an ordinary separation and purification of the polyclonal antibody.

### [Preparation of polyclonal antibody]

The polyclonal antibody of the present invention can be produced by a method known *per se* or a method according thereto. For example, the polyclonal antibody can be produced by preparing a complex of a peptide consisting of the above-mentioned antigenic determinant (E1 (E1') or E2 (E2')) and a carrier protein, immunizing a warm-blooded animal in the same manner as in the above-mentioned method of preparation of monoclonal antibody, recovering substances containing an antibody against the hapten from the immunized animal, and performing separation and purification of antibody.

Regarding the complex of a hapten and carrier protein used to immunize a warm-blooded animal, any kind of carrier protein can be crosslinked at any mixing ratio of carrier and hapten, as long as an antibody against the carrier-crosslinked immunized hapten is efficiently produced; for example, a method wherein bovine serum albumin, bovine thyroglobulin, KLH or the like is coupled at a ratio of about 0.1 to 20, preferably about 1 to 5, parts by weight per 1 part by weight of hapten, can be used.

For coupling of a hapten and a carrier protein, various condensing agents, for example, active ester reagents containing glutaraldehyde, carbodiimide, a maleimide active ester, a thiol group or a dithiopyridyl group, and the like can be used.

The condensation product, as is or along with a carrier or a diluent, is administered to a warm-blooded animal at a site permitting antibody production. To increase antibody productivity in this administration, Freund's complete adjuvant and Freund's incomplete adjuvant may be administered. The administration is normally conducted every 2 to 6 weeks, in a total of about 3 to 10 times.

A polyclonal antibody can be recovered from blood, ascites fluid, breast milk, egg and the like, of a warm-blooded animal immunized by the above-described method.

A measurement of the polyclonal antibody titer in the antiserum can be conducted in the same manner as the above-described measurement of antibody titer in the antiserum. Separation and purification of the polyclonal antibody can be conducted according to the same immunoglobulin separation and purification method as the above-described monoclonal antibody separation and purification.

Using the first antibody of the present invention and the second antibody obtained as mentioned above enables risk prediction of bone fracture and/or diagnosis of osteoporosis in a tested animal (e.g., human, horse, dog, cat, monkey, bovine, swine, sheep, goat, rabbit, mouse, rat, hamster, guinea pig, chicken and the like). More specifically, the present invention provides methods of risk prediction of bone fracture and/or diagnosis of osteoporosis by measuring a substance in a biological sample recognized by the first and the second antibody of the present invention (i.e., MEPE-derived molecule). "Diagnosis" herein not only refers to determining the presence or absence of onset, but also is used to include predicting whether or not the possibility of onset in future is high, evaluating the degree of progression of pathology in the tested animal where onset has already been known, and the like. Since the MEPE-derived molecule is recognized by the first and the second antibody of the present invention, the molecule contains at least antigenic determinants E1 (E1') and E2 (E2') of both antibodies of the present invention.

The biological sample used for the method of the present invention can be any, as long as it is derived from the tested animal; while examples can include body fluids (e.g., blood, plasma, serum, lymph fluid, synovial fluid, spermatic fluid, urine, sweat, tear and the like), various cells and tissue slices (e.g., articular cartilage and the like) and the like, blood, plasma, serum and the like are preferable since they are less invasive to the tested animal.

The method of the present invention is characterized by measuring the above-mentioned MEPE-derived molecule by sandwich method. The sandwich method in the present invention is used to encompass any methods comprising sandwiching the MEPE-derived molecule, which is a target, with the first antibody and the second antibody of the present invention to cause the first antibody-MEPE-derived molecule-the second antibody complex to form. Accordingly, the sandwich method of the present invention includes (1) the method wherein the biological sample is reacted with the first antibody (or the second antibody) of the present invention insolubilized on the carrier and the second antibody (or the first antibody) of the present invention in a free form simultaneously or sequentially, after which the amount of the first antibody-MEPE-derived molecule-the second antibody complex formed on the carrier is measured (heterogeneous method), and (2) the method wherein the biological sample is reacted with the first and the second antibody of the present invention simultaneously or sequentially, and the amount of the first antibody-MEPE-derived molecule-the second antibody complex formed in the solution is measured (homogeneous method).

In the heterogeneous method (narrowly-defined sandwich method), the antibody in the free state (hereinafter to be referred sometimes as liquid-phase antibody) is generally labeled with a suitable label. As examples of the labeling agent, a radioisotope, an enzyme, a fluorescent substance, a luminescent substance and the like can be used. As examples of the radioisotope, [¹²⁵I] , [¹³¹I] , [³H] , [¹⁴C] and the like can be used. As the enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-avidin system can also be used for binding of a liquid phase antibody and a labeling agent.

In insolubilizing the antigen or antibody, physical adsorption may be used, and a chemical bond in common use to insolubilize or immobilize a protein or an enzyme or the like, may also be used. As the carrier, insoluble polysaccharides such as agarose, dextran and cellulose, synthetic resins such as polystyrene, polyacrylamide and silicone, glass and the like can be mentioned.

In the sandwich method, the MEPE-derived molecule in the biological sample can be quantified by reacting the biological sample with the insolubilized first antibody (or the second antibody) of the present invention (hereinafter to be referred sometimes as solid phase antibody)(the first reaction), further reacting the biological sample with the labeled second antibody (or the first antibody) of the present invention (liquid phase antibody)(the second reaction), followed by separating the solid phase and the liquid phase (B/F separation) to remove the unreacted labeled liquid phase antibody and the like, and measuring the labeled amount on the insolubilizing carrier. The first reaction and the second reaction can be performed in the reverse order, and can be performed simultaneously or at different times.

In a more preferable embodiment of the present invention, as the solid phase antibody, the first antibody of the present invention is selected, and as the liquid phase antibody, the second antibody of the present invention is selected.

The sandwich complex can be also detected by surface plasmon resonance (SPR) method using a BIACORE (registered trade mark) chip and the like as the carrier, or by mass spectrometry method using a protein chip (manufactured by Ciphergen Biosystems Inc.) and the like; in these cases, the free antibody need not to be labeled.

Meanwhile, examples of the homogeneous method include, for example, (i) the method wherein one of the first and the second antibody of the present invention is labeled with a fluorescent substance and another is labeled with a quenching substance, the biological sample is reacted with these labeled antibodies in liquid phase, and the decrease in fluorescence released by the fluorescent substance is measured, (ii) the method wherein one of the first and the second antibody of the present invention is labeled with the first fluorescent substance (excitation wavelength λ₀; fluorescence wavelength λ₁) and another is labeled with the second fluorescent substance (excitation wavelength λ₁; fluorescence wavelength λ₂), the biological sample is reacted with these labeled antibodies in liquid phase, the reaction mixture is excited with light of wavelength λ₀, and the fluorescence of wavelength λ₂ is detected, and the like.

As the fluorescent substance used in the method mentioned in (i) above, for example, FAM, VIC and the like can be mentioned, and as the quenching substance, TAMRA, DABCYL, DABSYL, SYBR (registered trademark) Green I and the like can be mentioned.

As the combination of the first and the second fluorescent substances of the method mentioned in the (ii) above, for example, fluorescamine and dichlorotriazine fluorescein, fluorescein and rhodamine B, R-phycotriene and allophycocyanin, fluorescein and rhodamine X, fluorescein and Texas Red or Malachite Green and the like can be mentioned.

In applying these individual immunological measurement methods to the quantitation of the MEPE-derived molecule, it is unnecessary to set special conditions, procedures and the like. Making ordinary technical considerations for those skilled in the art to the ordinary conditions and procedures in each method, a measurement system for a MEPE-derived molecule can be constructed. For details of these general technical means, compendia, books and the like can be referred to. For example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.

Using the first and the second antibody of the present invention as described above, the MEPE-derived molecule can be quantified at high sensitivity.

When the first and the second antibody of the present invention are used, the level of the MEPE-derived molecule in the biological sample varies greatly among individual animals tested, and some individuals offer a level of the detection limit or below. Unexpectedly, in the assay system, the population from which the biological sample, in which an MEPE-derived molecule is not more than the detection limit, derives shows a significantly higher frequency of bone fracture compared to the population from which the biological sample, in which the MEPE-derived molecule was detected, derives.

Accordingly, it is possible to determine that the tested animal, wherein the MEPE-derived molecule is not detected or the level thereof is low, has high risk of future bone fracture, and/or is highly likely to be affected with osteoporosis or to be affected therewith in future. It is also possible to evaluate the degree of the progression of osteoporosis by monitoring the variation of the concentration of MEPE-derived molecule in the tested animal that has already been diagnosed to be affected with osteoporosis.

The present invention also provides a kit for risk prediction of bone fracture and/or diagnosis of osteoporosis, containing the first and the second antibody of the present invention. The kit can further contain other constituents suitable for practicing the above-mentioned method of the present invention, for example, reaction buffer, lavage fluid, carrier for insolubilization, labeling agent, MEPE preparation and the like.

When base, amino acid etc. are to be indicated with abbreviations in the specification and drawings, the abbreviations are adopted from IUPAC-IUB Commission on Biochemical Nomenclature or those commonly used in the art. For example, the following abbreviations are used. When an optical isomer is capable of existing with respect to the amino acids, the L-form is represented unless otherwise specified.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA:: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDTA:: Ethylenediaminetetraacetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyroglutamic acid
- Me:: Methyl group
- Et:: Ethyl group
- Bu:: Butyl group
- Ph:: Phenyl group
- TC:: Thiazolidine-4(R)-carboxamide group

Substituents, protecting groups and reagents frequently mentioned herein are represented by the symbols shown below.
- Tos:: p-Toluenesulfonyl
- CHO:: Formyl
- Bzl:: Benzyl
- Cl₂Bzl:: 2, 6-Dichlorobenzyl
- Bom:: Benzyloxymethyl
- Z:: Benzyloxycarbonyl
- Cl-Z:: 2-Chlorobenzyloxycarbonyl
- Br-Z:: 2-Bromobenzyloxycarbonyl
- Boc:: t-Butoxycarbonyl
- DNP:: Dinitrophenol
- Trt:: Trityl
- Bum:: t-Butoxymethyl
- Fmoc:: N-9-Fluorenylmethoxycarbonyl
- HOBt:: 1-Hydroxybenztriazole
- HOOBt:: 3,4-Dihydro-3-hydroxy-4-oxo-1,2,3-benzotriazine
- HONB:: 1-Hydroxy-5-norbornane-2,3-dicarboximide
- DCC:: N, N'-Dicyclohexylcarbodiimide

The present invention is hereinafter described in more detail by means of the following Examples, by which, however, the invention is not limited by any means. The operations of gene recombination using Escherichia coli were performed according to the method described in Molecular cloning, 2nd ed., J. Sambrook et al., Cold Spring Harbor Lab. Press (1989).

The anti-MEPE antibody-producing hybridomas obtained in Example below were deposited at the International Patent Organism Depositary (IPOD), National Institute of Advanced Industrial Science and Technology (AIST Tsukuba Central 6, 1-1-1 Tsukuba West, Tsukuba, Ibaraki 305-8566, Japan), as the following deposit numbers on April 26, 2005.
Hybridoma Cell
ME-12C9.2: FERM BP-10328
ME-16F4.3: FERM BP-10329

### Example 1 Cloning of cDNA encoding Matrix Extracellular Phospho-glycoprotein (MEPE)

A cDNA library derived from tumors of OHO patients was prepared as in the following. Specifically, a total RNA was obtained from a paranasal sinus tumor tissue of OHO patients according to phenol-chloroform-isoamyl alcohol, LiCl precipitation method (PLANT CELL PHYSIOLOGY 36: 85-93 (1995)). About 6 µg of poly(A)⁺ RNA was obtained from the extracted total RNA (about 480 µg) using Oligotex(dT)₃₀-Super (Takara Shuzo Co., Ltd.).

The cDNA library was prepared according to the method of Gubler and Hoffman. The first strand DNA was synthesized by using poly(A)⁺ RNA (2.5 µg), Oligo(dT)₁₈-linker primer
((GA)₁₀ACGCGTCGACTCGAGCGGCCGCGGACCG(T)₁₆) (SEQ ID NO: 4)
containing *Xho* I site, 5-methyl dCTP, dATP, dGTP, dTTP, RAV-2 reverse transcriptase and Superscript II reverse transcriptase. RNase H, DNA polymerase I and dNTP mixture were reacted thereto to synthesize the second strand DNA. The second strand DNA was blunt-ended, and was ligated with EcoR I-Not I-BamH I adapter (Takara Shuzo Co., Ltd.) using T4 DNA ligase. This was cleaved with restriction enzyme *Xho* I, followed by removal of a low molecular weight DNA using a spin column and ligation with λ ZAPII (*EcoR* I-Xho I cleaved) (STRATAGENE) using T4 DNA ligase. The thus obtained λ phage vector was incorporated into a λ phage precursor protein using an in vitro packaging kit (Stratagene) to give a λ phage library. The titer of the library was measured by using Escherichia coli XL1 Blue MRF' as a host. The titer of the primary λ phage library was 1.0×10⁶ pfu/ml.

The primary λ phage library was amplified at 4°C overnight by using Escherichia coli XL1 Blue MRF' as a host. About 5×10⁴ plaques were formed per one dish (diameter 150 mm) and SM buffer (10 ml) was layered thereon. Dimethyl sulfoxide was added to the recovered SM buffer to a final concentration of 7% and preserved at -80°C. The titer of the amplified λ phage library was not less than 1.0×10⁹ pfu/ml.

Cloning of MEPE cDNA was performed according to PCR method. Considering that the full-length cDNA of phosphatonin (MEPE) was not obtained since the phosphatonin gene sequence described in international publication (WO 99/60017) of international patent application by Rowe P.S.N. did not contain the N-terminal methionine residue and a secretion signal sequence, the λ phage library derived from tumors of OHO patients prepared above was used as a template; as primers, a synthetic oligo-DNA of SEQ ID NO: 5, which is present in 5' side of the multi-cloning site of pBluescript SK(+/-), which is the host vector used for the cDNA library, was used as a forward primer, and a synthetic oligo-DNA of SEQ ID NO: 6, which is present in 3' side of the stop codon of the phosphatonin gene sequence described in international publication (WO 99/60017) of international patent application by Rowe P.S.N., was used as a reverse primer.
5'-GGAAACAGCTATGACCATG-3' (SEQ ID NO: 5)
5'-TCAGGTGGCTCTCCTCTACATCAACTCACA-3' (SEQ ID NO: 6)

The PCR reaction was carried out by adding TaKaRa ExTaq DNA polymerase (Takara Shuzo Co., Ltd.) to the template and the primers mentioned above, and using a thermal cycler (GeneAmp PCR System, PE Applied Biosystems) under the conditions of 1 cycle at 95°C for 3 min, 30 cycles at 95°C for 45 sec, 58°C for 45 sec and 72°C for 3 min, 1 cycle at 72°C for 5 min, and letting stand at 4°C.

The obtained amplified fragment was subjected to an electrophoresis using 1% agarose gel, and the PCR products in a main band was extracted and purified, inserted into pCRII-TOPO vector (Invitrogen) using a TOPO TA cloning kit (Invitrogen) and introduced into Escherichia coli TOP10 strain.

Plasmid DNA was extracted from the obtained transformant, subjected to PCR reaction using BigDye Terminator Cycle Sequence Ready Reaction Kit (PE Applied Biosystems), and the base sequence of the cDNA fragment was determined by ABI PRISM^{™} 377 DNA sequencer (PE Applied Biosystems).

Although the insert sequence of the plasmid DNA retained by the obtained clone #1 had a base sequence (1713 bases) containing essentially the same base sequence as the base sequence (1290 bases) encoding Val (1st) to Asp (430th) of the phosphatonin gene sequence described in international publication (WO 99/60017) of international patent application by Rowe P.S.N., and encoded phosphatonin protein consisting of 525 amino acids, the insert sequence was different by several bases from the phosphatonin gene sequence described in international publication (WO 99/60017) of international patent application by Rowe P.S.N. and the insert sequence of plasmid DNA retained by simultaneously obtained other clones. To determine the true MEPE gene sequence, therefore, PCR was performed using Pyrobest DNA polymerase with a higher fidelity. The λ phage library prepared in the above, which was derived from tumor of OHO patients, was used as a template, and as primers, a synthetic oligo DNA of SEQ ID NO: 7, which starts from the 5' side of initiation codon ATG of the above-mentioned clone #1 was used as a forward primer and a synthetic oligo DNA of SEQ ID NO: 6 was used as a reverse primer.
5'-CTCAAAGATGCGAGTTTTCTGTGTGGGA-3' (SEQ ID NO: 7)

The PCR reaction was carried out using a thermal cycler (GeneAmp PCR System, PE Applied Biosystems) under the conditions of 30 cycles at 98°C for 10 sec, 56°C for 45 sec and 72°C for 3 min, and letting stand at 4°C.

The obtained amplified fragment was subjected to a gel electrophoresis using 1% agarose gel, and the PCR products in a main band was extracted and purified, inserted into pCR-Blunt vector (Invitrogen) using a Zero Blunt PCR Cloning Kit (Invitrogen) and introduced into Escherichia coli competent cell TOP10 strain.

Plasmid DNA was extracted from the obtained transformed bacteria, subjected to PCR reaction using BigDye Terminator Cycle Sequence Ready Reaction Kit (PE Applied Biosystems), and the base sequence of cDNA fragment was determined by ABI PRISM^{™} 377 DNA sequencer (PE Applied Biosystems). As a result, a transformant: Escherichia coli TOP10/pCR-PHOS (clone #9) containing plasmid pCR-PHOS retaining a DNA encoding the full-length of MEPE protein was obtained.

The plasmid DNA retained by the obtained TOP10/pCR-PHOS (clone #9) had a base sequence (1662 bases) represented by SEQ ID NO: 3 containing the base sequence (1575 bases) represented by SEQ ID NO: 1, and encoded phosphatonin (MEPE) protein consisting of 525 amino acids represented by SEQ ID NO: 2. The molecular weight of the protein of the present invention (including signal peptide) was 58.4 kDa as deduced from the amino acid sequence.

The base sequence represented by SEQ ID NO: 1 had a base sequence essentially the same as a base sequence (1290 bases) encoding Val (1st) to Asp (430th) of phosphatonin described in international publication (WO 99/60017) of international patent application by Rowe P.S.N. and had a novel base sequence (285 bases) encoding 95 amino acid sequence starting with Met in the 5' region thereof. In the sequence common to base sequence represented by SEQ ID NO: 1 and base sequence of phosphatonin described in international publication (WO 99/60017) of international patent application by Rowe P.S.N., only the 286th base was different (Rowe P.S.N. publication: G¹→SEQ ID NO: 1: C²⁸⁶), along with which the amino acid sequence represented by SEQ ID NO: 1 was different in one residue (Rowe P.S.N. publication: Val¹→SEQ ID NO: 1: Leu⁹⁶). The amino acid sequence of the protein consisting of 525 amino acids represented by SEQ ID NO: 2 and the base sequence represented by SEQ ID NO: 1 encoding same were completely identical to the amino acid sequence from Met (1st) to Asp (525th) of phosphatonin described in international publication (WO 01/32878) of international patent application by Rowe P.S.N. and the base sequence (1,575 bases) encoding same. Example 2 Secretory expression of recombinant MEPE-FLAG in insect cell

A vector was transformed into JM109 competent cell, wherein the vector was prepared by ligating a-MEPE (phosphatonin)-FLAG structural gene, which had been cleaved with BamH I and *Sal* I from phosphatonin (MEPE)-FLAG adduct animal cell expression vector (pT-PHOSF-11, Fig. 4) described in Example 6 in international publication (WO 01/98485) of international patent application by Yamada T., and pFastBac1 vector treated with the same restriction enzyme. Donor plasmid DNA (pFastBac-PHOSF) was prepared from the obtained transformant (JM109/pFastBac-PHOSF), and the base sequence thereof was confirmed. The donor plasmid DNA was added to a competent cell (MAX Efficiency DH10Bac^{™}) (Invitrogen^{™}Life technologies) containing Bacmid and a helper, and antibiotic selection was performed to give the transformant. Furthermore, genetic recombinant Bacmid DNA (Bacmid-PHOSF) was prepared from the transformant, transfected into insect cell strain (SF⁺) in the presence of CellFECTIN reagent to prepare a baculovirus stock solution for expression of recombinant MEPE-FLAG. The insect cell strain (SF⁺) was infected with the solution, and secretory expression of recombinant MEPE-FLAG protein, molecular weight 65 kDa, in culture supernatant was confirmed by Western blot using separately-prepared anti-MEPE rabbit polyclonal antibody and HRPO-labeled anti-rabbit IgG goat antibody.

### Example 3 Preparation of recombinant MEPE-FLAG in insect cell expression

The insect cell strain (SF⁺) was incubated in 6 L scale, and the baculovirus stock solution obtained in Example 2 was added thereto to induce secretion expression of the recombinant MEPE-FLAG. The level of the recombinant MEPE-FLAG protein in the culture supernatant was monitored over time by Western blot, and the culture was stopped at the time point when the expression level was at the maximum.

Purification of the recombinant MEPE-FLAG in insect cell expression was performed according to the method described in Example 7 in international publication (WO 01/98485) of international patent application by Yamada T. Each 1 L of the culture supernatant after the baculovirus infection of the insect cell was adsorbed to an anti-MEPE rabbit polyclonal antibody column (5 ml), which had been equilibrated with 50 mM Tris-HCl (pH 8.0), at a flow rate of 4.5 ml/min. This column was washed with Tris-HCl (pH 8) containing 0.5 M NaCl, after which the recombinant MEPE-FLAG protein bound to the column was eluted with 0.1 M glycine-HCl (pH 3.0) containing 0.5 M NaCl, and 1 M Tris-HCl (pH 8.0) was added to neutralize the eluate. The eluate was concentrated and substituted with PBS using an ultrafiltration membrane (Vivaspin 20: molecular weight cut off 10 kDa) (Sartorius Co., Ltd.), and the product was used as the final preparation. The series of the operations was repeated 6 times, and the recombinant MEPE-FLAG preparation (18 mg) was obtained from 6 L of the culture supernatant of the baculovirus-infected insect cell. SDS-polyacrylamide gel electrophoresis (SDS-PAGE) was performed in order to measure the purity of the thus-obtained MEPE-FLAG preparation by insect expression. The preparation was mixed with a sample buffer supplemented with 100 mM DTT [Laemmli, Nature, 227: 680 (1979)], heated at 95°C for 1 min, and subsequently subjected to a Multigel 10/20 (Daiichi Pure Chemicals Co., Ltd.) to perform electrophoresis. The gel after the electrophoresis was stained with Coomassie·brilliant blue, resulting in observation of a protein with an almost single band at about 70 kDa (Fig. 2, Lane 2).

The N-terminal amino acid sequence of the purified MEPE-FLAG preparation was determined by using a gas-phase protein sequencer (Applied Biosystems model 492). As a result, the N-terminal amino acid sequence of the preparation was identical to the sequence (APTFQPQTE) wherein the signal peptide sequence was deprived from the N-terminal amino acid sequence of MEPE deduced from the base sequence.

### Example 4 Production of anti-MEPE mouse monoclonal antibody

Eight BALB/c mice (8-week-old, female; CLEA Japan) were immunized subcutaneously and intradermally with an emulsion prepared by mixing the recombinant MEPE-FLAG preparation by insect cell expression prepared in Example 3 (1 mg/ml PBS solution) and Freund's complete adjuvant at equal volume, at 40 µg/animal. At the second immunization or later, the mice were boostered every 2 weeks with the emulsion prepared from the same MEPE-FLAG preparation and Freund's incomplete adjuvant as in the same manner.

At time points before start of the immunization and 1 week after the third immunization, all of the each mouse was subjected to blood collection from ocular fundus under ether anesthesia to obtain antiserum, and serum antibody titer was determined by the below-described ELISA. Specifically, the mouse antiserum was serially diluted with PBS containing 0.2% bovine serum albumin (BSA), and the diluted solutions were added to an immunoplate (Nunc) coated with the recombinant MEPE-FLAG and blocked with 2% BSA-containing PBS, at 100 µl/well, and reacted at room temperature for 2 hr. This plate was washed 4 times with 0.05% Tween 20-containing PBS, added with HRPO-labeled anti-mouse IgG goat antibody (Chemicon) diluted 3,000-times with 0.2% BSA-containing PBS at 100 µl/well, and further reacted at room temperature for 2 hr. The plate was washed 6 times with 0.05% Tween 20-containing PBS, added with 3,3',5,5'-tetramethyl benzidine (TMB) solution (TMB Peroxidase EIA Substrate Kit; Bio-Rad Laboratories) at 100 µl/well, allowed to develop color at room temperature for 5 min, and added with 1 N sulfuric acid (Wako Pure Chemical Industries) at 100 µl/well to stop the enzyme reaction. Absorbance (450 nm) of each well was measured using a plate reader (Multiskan BICHROMATC; Labsystems), and a graph with the horizontal axis being serum dilution rate and the vertical axis being absorbance was created to compare the serum antibody titers.

The top 2 mice wherein sufficient elevation in the serum antibody titer had been confirmed as in the manner described above were injected through tail vain with the recombinant MEPE-FLAG at 10 µg/animal, and were subjected to death from exsanguination 3 days later to isolate the spleens. Mouse spleen cells prepared by breaking these spleens into flakes and mouse myeloma cells (P3X63Ag8U.1) which had been previously adapted to a culture in a serum-free medium (S-clone SF-B; Sanko Junyaku Co., Ltd.) were mixed at the ratio of 5:1, and fused together by using polyethylene glycol (PEG) 1,500 (Roche Diagnostics K.K.). The cell fusion was performed according to the manual attached with the reagent. The cells after the fusion were sown on a 96-well culture plate with a serum-free medium for cloning (S-clone CM-B; Sanko Junyaku Co., Ltd) at 1×10⁵ splenocytes/100 µl/well, incubated in a CO₂ incubator (5% CO₂, 37°C) for 1 day, and subsequently added with 0.1 mM hypoxanthine -0.4 µM aminopterine-0.016 mM thymidine (HAT) addition S-clone CM-B medium at 100 µl/well, the incubation was continued in the CO₂ incubator, and 3/4 of the culture supernatant was replaced with the same fresh HAT addition medium twice every 3 days.

During 7th to 14th day of the incubation, the culture supernatant in wells in which growth of hybridoma colony had been observed was subjected to the aforementioned ELISA for measurement of the anti-MEPE mouse antibody to give a great number of positive wells. IgG antibody-producing hybridomas with high reactivity were selected from the positive wells and cloned by limiting dilution method, and 8 kinds of anti-MEPE mouse monoclonal antibody-producing hybridomas [ME-2D11.9 (IgG₁/κ), ME-4A2.2 (IgG₁/κ), ME-9D2.15 (IgG₁/κ), ME-9H6.8 (IgG₁/κ), ME-10E4.2 (IgG₁/κ), ME-12C9.2 (IgG₂ₐ/κ), ME-12E2.1 (IgG₁/κ), ME-16F4.3 (IgG₁/κ)] were established, which were freeze-preserved. Subclasses of the monoclonal antibodies produced by each of the hybridomas were determined using IsoStrip Mouse Monoclonal Antibody Isotyping Kit (Roche Diagnostics K.K.).

The above-mentioned 8 kinds of hybridomas were incubated (500 ml) with a serum-free medium (S-clone SF-B) and centrifuged (1,200 rpm × 5 min), and the supernatant fluid was further filtrated with a 0.2 µm filter to prepare culture supernatants containing the monoclonal antibody. These culture supernatants were mixed with equal volume of Protein A MAPS II binding buffer, and adsorbed to Protein A Sepharose FF (16 mm ID×25 mm, 50 µm, 5 ml)(Amersham Biosciences K.K.) which had been equilibrated with the same buffer. This column was washed sufficiently with Protein A MAPS II binding buffer, after which IgG fraction was eluted with Protein A MAPS II elution buffer. The obtained fraction was neutralized with 1 M Tris-HCl (pH 8.0), and subsequently dialyzed against PBS at 4°C overnight to give 8 kinds of purified anti-MEPE mouse monoclonal antibody preparations. The MEPE-binding activity of these antibodies was confirmed by the aforementioned ELISA (Fig. 3).

### Example 5 ELISA for measurement of MEPE-derived antigen concentration in plasma

Various combinations of the 8 kinds of the anti-MEPE mouse monoclonal antibodies prepared in Example 4 were examined; as a result, a sandwich ELISA for measurement of MEPE-derived antigen concentration in human plasma was established with MAB-ME-16F4.3 as a solid phase antibody and with biotinylated MAB-ME-12C9.2 as a liquid phase antibody.

The biotinylated MAB-ME-12C9.2 was prepared by reacting a PBS solution of the anti-MEPE mouse monoclonal antibody (MAB-ME-12C9.2) prepared in Example 3 with 20-fold molar amount of EZ-link^{™}Sulfo-NHS-LC-Biotin (Pierce Biotechnology, Inc.; #21335) at room temperature for 1 hr, and dialyzing the reaction mixture against PBS overnight.

Procedure of an optimized sandwich ELISA is described below. MAB-ME-16F4.3 was diluted with 0.05% sodium azide-containing 50 mM sodium carbonate/sodium bicarbonate buffer (pH 9.6) to a concentration of 10 µg/ml, and the diluted solution was added to a 96-well immunoplate (Nunc) at 100 µl/well and reacted at room temperature for 5 hr. After removal of the reaction solution, a 4-fold diluted solution of BlockAce (Dainippon Pharmaceutical Co., Ltd.) was added to the immunoplate at 200 µl/well and reacted at 4°C overnight to prepare a plate for ELISA. The concentration of the recombinant MEPE-FLAG by insect cell expression, prepared in Example 3, was determined by amino acid composition analysis, and the preparation was diluted to 10 µg/ml with 10% BlockAce-containing PBS, subdivided into small portions, and preserved at -80°C, to prepare a standard stock solution. A MEPE standard solution was prepared by thawing the standard stock solution when in use, and further diluting the solution with 10% BlockAce-containing PBS. Human plasma prepared from bloods containing ethylenediamine tetraacetate (EDTA) which were taken from healthy volunteers and postmenopausal female patients who gave an informed consent, subdivided into small portions, and preserved at -80°C, were used.

The plate for ELISA prepared above was washed twice with 0.05% Tween 20-containing PBS, and the MEPE standard solution or a human plasma sample diluted with 10% BlockAce-containing PBS was added at 100 µl/well, and it was reacted at room temperature for 2 hr. After washing the plate 4 times with 0.05% Tween 20-containing PBS, the aforementioned biotin-labeled MAB-ME-12C9.2 solution diluted with 10% BlockAce-containing PBS at 1.2 µg/ml, was added at 100 µl/well and it was reacted at room temperature for 2 hr. After further washing the plate 4 times with 0.05% Tween 20-containing PBS, HRPO-Avidin D (Vector Laboratories) diluted to 1 µg/ml with 10% BlockAce-containing PBS was added at 100 µl/well, and it was reacted at room temperature for 2 hr. Furthermore, the plate was washed 6 times with 0.05% Tween 20-containing PBS, and a TMB solution (TMB Peroxidase EIA Substrate Kit; Bio-Rad Laboratories) was added at 100 µl/well to allow color development at room temperature for 30 min, and then 2 N sulfuric acid (Wako Pure Chemical Industries) was added at 100 µl/well to stop the enzyme reaction. Absorbance (450 nm) of each well was measured using a plate reader (Multiskan BICHROMATC; Labsystems), and MEPE level in human plasma was quantified using the analytical curve plotted based on the MEPE standard solution (Fig. 4). A coefficient of intradaily variation and a coefficient of interdaily variation in the present ELISA were as low as 5.8-7.3% and 7.0-8.0%, respectively (Table 1).

**Table 1 Accuracy of measurement of MEPE by the ELISA**

| Sample | Intradaily assay | | Interdaily assay | |
|---|---|---|---|---|
| | MEPE (ng/ml)^{a)} | CV (%)^{b)} | MEPE (ng/ml) | CV (%) |
| A | 3.13±0.18 | 5.8 | 2.91±0.20 | 7.0 |
| B | 1.26±0.08 | 6.6 | 1.10±0.09 | 8.0 |
| C | 0.61±0.05 | 7.3 | 0.53±0.03 | 7.4 |

| | | | | |
|---|---|---|---|---|
| a) The values represent the average of 5 experiments ±S.D.. b) CV=100×S.D./average | | | | |

### Example 6 Risk evaluation of vertebral body fracture by measurement of MEPE-derived antigen in plasma from postmenopausal osteoporosis patients

MEPE-derived antigen in plasma from 73 postmenopausal females not having received a bisphosphonate administration (average±standard deviation (SD) 71.6±11.3 year-old, 38.0-94.4 year-old) was measured with the sandwich ELISA described in Example 5. As a result, while the MEPE-derived antigen was significantly detected in plasma from 18 out of 73 females, the levels of the MEPE-derived antigen in plasma from 55 females (75.3%) were not more than the measurement limit of the present ELISA (the detection limit was defined as average +2SD (=0.59 ng/ml) in samples wherein the measured value by the ELISA was not more than 0). Comparison between the 2 groups exhibited no significant difference in age, height, BMI, bone metabolism marker [urinary deoxypyridinoline (DPD) and bone alkaline phosphatase (BAP) (measured with osteolinks BAP and osteolinks Dpd (both Quidel Corp, Santa Clara, CA, USA), respectively], bone density of lumbus, femur or radius [measured with Dual Energy X-ray Absorptimetry (DPX-L, Lunar Co., Madison, WI)] between the 2 groups (Table 2). On the other hand, regarding the number of vertebral body fracture, the patients group in which MEPE-derived antigen level in plasma detected by the ELISA was not less than the detection limit had significantly smaller number of vertebral body fracture compared to the patients group in which the same kind of the measurement value was not more than the detection limit (0.27±0.59 vs 1.35±2.91) (Table 2 and Fig. 5).

**Table 2**

| | ELISA value | | T-test of significant difference between the both groups |
|---|---|---|---|
| Measurement item | Less than the detection limit* | Not less than the detection limit | |
| Bone fracture number | 1.35±2.91 | 0.27±0.59 | 0.013 |
| Age | 72.8±10.4 | 67.6±13.3 | 0.100 |
| Height | 153±5.8 | 156±6.3 | 0.165 |
| Lumbus BMD t-score | -2.29±1.41 | -1.69±1.86 | 0.173 |
| BAP | 27.5±12.8 | 27.5±1.86 | 0.992 |
| Urinary DPD | 6.18±3.52 | 5.78±2.52 | 0.661 |
| BMI | 21.1±2.84 | 21.1±2.28 | 0.990 |

| | | | |
|---|---|---|---|
| * Detection limit: 0.59 ng/ml | | | |

As described above, the result in which there was significant difference in the number of bone fracture between the two groups which were divided each other by MEPE-derived antigen level in plasma detected by the present ELISA and had no difference in bone density and bone metabolism marker shows the possibility that the MEPE-derived antigen is clinically involved in bone strength and is significantly associated with vertebral body fracture, and contributes to bone strength via bone substance, independently of bone mass. Therefore, the possibility can be considered that the present ELISA can be used as an index of bone substance and bone strength, and a system of risk evaluation of bone fracture, which is different from a marker of bone density or bone metabolic turnover. Example 7 Recognition site analysis of anti-MEPE mouse monoclonal antibody (1)

In order to clarify the recognition sites of the 2 kinds of anti-MEPE mouse monoclonal antibodies (MAB-ME-12C9.2, MAB-ME-16F4.3) used for the ELISA mentioned in Example 5 and Example 6, 3 kinds of Escherichia coli expression vectors were constructed by deleting a sequence at their C-terminal side of 3 kinds of MEPE fragments (MEPE (Δ507-525), MEPE (Δ331-525) and MEPE (Δ241-525); which means that the region specified by the amino acid number corresponding to the amino acid sequence shown by SEQ ID NO: 2 was deleted) and adding a FLAG peptide to each of the C-terminals, and these vectors were transformed into Escherichia coli competent cell BL21(DE3) to obtain each of the transformants.

The obtained transformants were subjected to shaking culture at 37°C for 3 hr in a 250 ml side-arm flask containing 30 ml of LB medium (1% peptone, 0.5% yeast extract, 0.5% sodium chloride) supplemented with 100 µg/ml of ampicillin. At the time point when the turbidity of the culture medium reached about 150 Klett unit, isopropyl-β-D-thiogalactopyranoside (IPTG) with the final concentration of 0.4 mM was added to the flask, and the culture was continued for additional 4 hr to obtain a culture medium containing Escherichia coli strain expressing the MEPE mutein. A portion of the culture medium was subjected to SDS-PAGE, followed by protein staining with Coomassie·brilliant blue and Western blot using an anti-FLAG antibody to confirm the expression of the object MEPE mutein.

Mature-type recombinant MEPE-FLAG and the culture medium of the above-mentioned 3 kinds of MEPE mutein-expressing Escherichia coli strains (2.5 µl) were mixed with equal volume of a sample buffer supplemented with 100 mM DTT, heat-treated at 95°C for 5 min, and subsequently subjected to a Multigel 10/20 (Daiichi Pure Chemicals Co., Ltd.) to perform SDS-PAGE. The migrated proteins in a gel after the electrophoresis were transferred to a PVDF membrane, and a Western blot was performed using MAB-ME-12C9.2, MAB-ME-16F4.3 and separately-prepared anti-MEPE rabbit polyclonal antibody, and HRPO-labeled anti-mouse IgG goat antibody (Chemicon) and HRPO-labeled anti-rabbit IgG goat antibody (Chemicon). While plural bands were detected for each mutein, a main band at the top of the membrane corresponding to the molecular size deduced from the amino acid sequence and stained with an anti-FLAG antibody was judged to be the object MEPE mutein, and bands at the lower molecular weight side were judged to be degradation products.

The anti-MEPE rabbit polyclonal antibody bonded both to the mature-type recombinant MEPE-FLAG and to the above-mentioned 3 kinds of MEPE mutein. On the other hand, MAB-ME-12C9.2 and MAB-ME-16F4.3 bonded to the mature-type recombinant MEPE-FLAG, MEPE (Δ507-525) and MEPE (Δ331-525), but didn't bond to MEPE (Δ241-525) (Fig. 6), which suggests that an epitope of these monoclonal antibodies be present in Tyr²⁴¹-Val³³⁰ in MEPE molecule.

### Example 8 Recognition site analysis of anti-MEPE mouse monoclonal antibody (2)

The epitope scanning method was performed in order to specify the epitope of the monoclonal antibodies identified in Example 7 in more detail. The outline is described below. Partial peptides consisting of 12 amino acid residues, which were chosen by shifting such that each 3 amino acid residues from N-terminal side overlap with MEPE (Tyr²⁴¹-Val³³⁰), were solid phase synthesized on a Whatman 50 cellulose membrane in spots (Fig. 7A) (Custom SPOTs, SIGMA Genosys). The synthesized peptides were immobilized to the membrane via a linker at their C-terminal side, and the N-terminal amino groups were acetylated. This membrane was immersed in methanol for 5 min, and subsequently washed 3 times with a 50 mM Tris-HCl buffered saline (pH 8.0) (TBS) containing 137 mM NaCl and 2.7 mM KCl at room temperature for 10 min. This membrane was shaken and blocked with a blocking agent (Genosys, Cat. No. SU-07-250) diluted 10 times with TBS at room temperature for 5 hr. This membrane was reacted in an anti-MEPE monoclonal antibody (MAB-ME-16F4.3) solution (0.5 µg/ml) diluted with the same blocking agent which had been diluted 20 times with 0.05% Tween 20-containing TBS (TTBS) at room temperature for 3 hr, washed with TTBS at room temperature for 10min, and further reacted in a HRPO-labeled anti-mouse IgG(H+L) goat antibody (Bio-Rad Japan, Richmond, CA, Cat. No. 170-6516) solution diluted 20,000 times with the same blocking agent which had been diluted 20 times with TTBS at room temperature for 2 hr. This membrane was washed 3 times with TBS at room temperature for 5 min, added with a luminescence reagent for HRPO, ECL-Plus (Amersham Biosciences K.K., Cat. No. RPN2132), and luminescence spots were detected using a luminoimage analyzer (LAS-1000, Fujifilm) (Fig. 7B).

The SPOTs membrane used above was washed 3 times with distilled water at room temperature for 10 min, and subsequently treated 4 times with a recovery buffer (62.5 mM Tris-HCl (pH 6.7) containing 2% SDS and 100 mM 2-mercaptoethanol) at 50°C for 30 min to strip the primary antibody and the secondary antibody. This was washed sequentially with 10-fold concentrated phosphate-buffered saline (pH 7.2) and TTBS at room temperature for 20 min, 3 times each, and further washed 3 times with TBS at room temperature for 10 min. The thus recovered membrane was reacted with another anti-MEPE monoclonal antibody (MAB-ME-12C9.2) by the same method as mentioned above, and the luminescence spots were detected (Fig. 7B).

Peptide sequences corresponding to the thus obtained main luminescence spots were matched; as a result, it was assumed that the epitope of MAB-ME-16F4.3 was contained in the amino acid sequence of Ser²⁵³-Pro²⁶¹, and that the epitope of MAB-ME-12C9.2 was contained in the amino acid sequence of Thr²⁹⁸-Ile³⁰⁶, respectively (Fig. 7C).

### Industrial Applicability

The two kinds of anti-MEPE antibodies of the present invention can specifically detect-MEPE-derived molecules in a biological sample, which can be a diagnosis marker of osteoporosis; therefore, they are extremely useful for risk prediction of bone fracture and diagnosis of osteoporosis in animal.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."

This application is based on patent application No. 2005-269072 filed in Japan, and the contents disclosed therein are hereby entirely incorporated by reference. In addition, the contents disclosed in any publication cited herein, including patents and patent applications, are hereby incorporated in their entireties by reference, to the extent that they have been disclosed herein.

## Claims

1. An antibody of the following (a) or (b):
(a) mouse monoclonal antibody MAB-ME-16F4.3 (FERM BP-10329)
(b) an antibody capable of specifically recognizing the same amino acid sequence as that of an antigenic determinant of the antibody of the above-mentioned (a).

2. The antibody described in claim 1, wherein the antigenic determinant is present in the amino acid sequence shown by amino acid numbers 253-261 in the amino acid sequence shown by SEQ ID NO: 2.

3. An antibody of the following (a) or (b):
(a) mouse monoclonal antibody MAB-ME-12C9.2 (FERM BP-10328)
(b) an antibody capable of specifically recognizing the same amino acid sequence as that of an antigenic determinant of the antibody of the above-mentioned (a).

4. The antibody described in claim 3, wherein the antigenic determinant is present in the amino acid sequence shown by amino acid numbers 298-306 in the amino acid sequence shown by SEQ ID NO: 2.

5. A method for predicting the risk of bone fracture and/or diagnosis of osteoporosis, which comprises using the antibody described in claim 1 and the antibody described in claim 3.

6. The method described in claim 5, which comprises measuring a substance in a biological sample, wherein the substance is recognized by the antibody described in claim 1 and the antibody described in claim 3.

7. The method described in claim 6, which comprises measuring the substance by sandwich method.

8. The method described in claim 6, wherein the biological sample is blood, serum or plasma.

9. A kit for predicting the risk of bone fracture and/or diagnosis of osteoporosis, comprising the antibody described in claim 1 and the antibody described in claim 3.
